# EUROPEAN PATENT SPECIFICATION

(11) **EP 1 109 776 B1**
(45) Date of publication and mention of the grant of the patent: **04.06.2003**
(21) Application number: 98943507.8
(22) Date of filing: 02.09.1998
(51) Int. Cl.: C07C 69/653, C08F 20/30

(54) **BROMINATED MATERIALS**
BROMIERTE MATERIALIEN
MATERIAUX BROMES

(43) Date of publication of application: 27.06.2001
(73) Proprietor: MINNESOTA MINING AND MANUFACTURING COMPANY, St. Paul, Minnesota 55133-3427 (US)
(72) Inventor: OLSON, David, B., Marine on Saint Croix, MN 55047 (US)
(74) Representative: VOSSIUS & PARTNER
(86) International application number: US9818127
(87) International publication number: WO00014050

(56) References cited:
- PATENT ABSTRACTS OF JAPAN vol. 13, no. 39 (C-563), 27 January 1989 & JP 63 238103 A (MATSUSHITA ELECTRIC WORKS LTD), 4 October 1988
- PATENT ABSTRACTS OF JAPAN vol. 14, no. 363 (C-0746), 7 August 1990 & JP 02 133445 A (MATSUSHITA ELECTRIC WORKS LTD), 22 May 1990
- PATENT ABSTRACTS OF JAPAN vol. 11, no. 317 (C-452), 15 October 1987 & JP 62 106912 A (SHOWA DENKO KK), 18 May 1987
- DATABASE WPI Week 8725 Derwent Publications Ltd., London, GB; AN 87-174468 XP002100215 & JP 62 106050 A (SHOWA DENKO KK) , 16 May 1987

## Description

The invention relates to (alkyl,bromo)phenoxy alkyl (meth)acrylate monomers and polymers made therefrom.

Reactive chemical monomers can be used to prepare polymeric materials which have various properties and which are useful for various applications. As one example, monomers having optical properties can generally be used, alone or in combination with other reactive materials, to produce.useful products having a high index of refraction, and that are useful to control the flow and intensity of light. To continually improve such products, or the processes for preparing such products, there is an ongoing need to develop new and improved high index of refraction monomeric materials.

Some brominated aromatic (meth)acrylate monomers have been found to be useful as high index of refraction monomers. These monomers can exhibit desirable optical qualities, but generally tend to display relatively high melting points, and therefore exist as solids al temperatures near room temperature (e.g., in the range about 20 to 30°C). Often such known brominated monomers have melting points significantly above room temperature. In addition, polymerization of these monomers (by themselves or with other comonomers) can frequently lead to a polymer with a relatively high glass transition temperature (Tg) which can limit the range of application of such monomers

JP-A-63 238 103 describes a photo-setting resin composition obtained by blending a photo-setting resin (e.g. epoxy acrylate) with (A) a reactive bromine compound having a radically polymerizable reactive group and one or more bromine atoms in the molecule (e.g. 2,6-dibromo-4-isopropyl phenyl propylene glycol methacrylate) in an amount of preferably 5 to 80 PHR and (B) antimony pentoxide in an amount of preferably 0.5 to 15 PHR. The composition is said to be utilized for sealing electronic parts.

JP-A-02 133 445 describes an electrical laminate having improved flame retardency, wherein a thermosetting resin varnish (a) (e.g. epoxy resin varnish) is mixed with a radically polymerizable reactive bromine compound (b) having at least one bromine atom in the molecule (e.g. 2,6-dibromo-4-isopropyl phenyl propylene glycol methacrylate), and Sb₂O₅ (c) to obtain a component (B). A plurality of resin -impregnated bases (A) obtained by impregnating bases (e.g. woven glass cloth) with component (B) and drying them are laid upon each other and the assemblage is laminate-molded under applied heat and pressure.

JP-A-62 106 050 and JP-A-62 106 912 describe a polymerizable monomer expressed by formula I 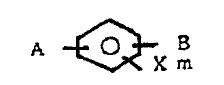 wherein group A and group B are mutually located at the o- or p-position and A is 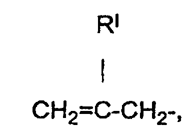 and B is 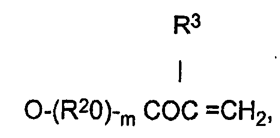 wherein X is Cl or Br and m and n are integers of 1 to 4.

It would be desirable to identify monomers useful to produce optical materials, where the monomers have physical properties including a relatively high index of refraction, a relatively low melting point in combination with a relatively low room temperature viscosity, and which can be used to prepare polymers (e.g., homopolymers or copolymers) having a relatively low Tg.

The invention provides (alkyl,bromo)phenoxy alkyl (meth)acrylate monomers according to claim 1. The term (alkyl, bromo) phenoxy alkyl (meth)acrylate is used herein to refer to chemical compounds comprising a (meth)acrylate, a phenoxy ring substituted with at least bromine and an alkyl group, and a divalent alkylene group connecting the (meth)acrylate to the phenoxy ring. Preferred monomers exhibit a relatively high index of refraction; i.e., at least 1.50. Preferred monomers also have a relatively low melting temperature; i.e., below about 60 degrees celsius (60°C), more preferably below 35°C. or 30°C, and most preferably exist as a liquid at or near normal room temperature (e.g., 25°C). In addition, preferred monomers have a relatively low room temperature viscosity, and can be polymerized, either alone or in combination with one or more other comonomers, to prepare polymers with a relatively low glass transition temperature (Tg), e.g., <50°C.

An aspect of the invention relates to (alkyl,bromo)phenoxy alkyl (meth)acrylate monomers having the general formula: 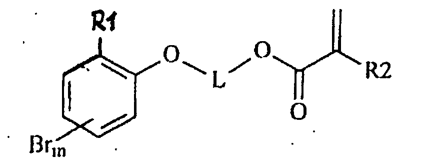 wherein m is from 1 to 4; R2 is hydrogen or methyl, R1 is a straight or branched alkyl having at least two carbon atoms, and L is a straight chain or branched alkylene.

Another aspect of the invention relates to a polymerizable composition containing an (alkyl,bromo)phenoxy alkyl (meth)acrylate monomer such as that defined directly above. The nolymerizable compositicm can further contain one or more other comonomer.

Yet another aspect of the invention relates to a polymer or polymeric material comprising a chemical segment having the formula: 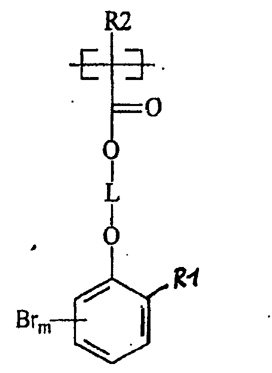 wherein m is from 1 to 4, R2 is -H or methyl, R1 is a straight or branched alkyl having at least two carbons, and L is a straight or branched alkylene. Such a polymer can be prepared by polymerization of the (alkyl,bromo)phenoxy alkyl (meth)acrylate monomer.

As used within the present description, "monomer" refers to a monomer on an individual (i.e., molecular) scale, and also to a composition of such monomers on a macroscopic scale such that the composition can he described as having a physical state of matter (e.g., liquid, solid, etc.) and physical properties (e.g., melting point, viscosity, glass transition temperature (of a polymeric form), and index of refraction).

"Index of refraction," or "refractive index," refers to the absolute refractive index of material (e.g., a monomer), which is understood to be the ratio of the speed of electromagnetic radiation in free space to the speed of the radiation in that material, with the radiation being of sodium yellow light at a wavelength of about 583.9 nanometer (nm). Index of refraction can be measured by known methods, and is generally measured using an Abbe Refractometer.

"Glass transition temperature," (Tg), is the temperature range over which a thermoplastic polymer changes from a brittle, glass state to a plastic state. Tg can be measured by methods known in the analytical chemistry art, such as the method described in the Examples section below.

"(Meth)acrylale" refers to both acrylate and methacrylate compounds.

Monomers of the invention are (alkyl, bromo) phenoxy alkyl (meth)acrylate monomers, according to claim 1, wherein the alkyl group includes at least two carbon atoms (also referred to herein as "the monomer" or "the brominated monomer," in both singular and plural forms). The (alkyl,bromo)phenoxy alkyl (meth)acrylate monomer can comprise a (meth)acrylate, a phenoxy ring substituted with substituents comprising bromine and an alkyl group, and a divalent alkylene group connecting the two.

The alkyl group can be straight or branched, and can preferably have from 2 to 12 carbon atoms, more preferably from 3 to 12 carbon atoms. The size, position, and structure of the alkyl group are believed to affect properties of the monomer and polymers prepared therefrom, including the refractive index and viscosity of the monomer, and the refractive index and Tg of a polymer made from the monomer. For example, relatively larger or more branched alkyl groups can provide monomers capable of being polymerized to polymers having relatively lower glass transition temperatures, compared to otherwise similar monomers having fewer carbon atoms or less branching. Additionally, a relatively larger alkyl group can result in a monomer or polymer having a relatively lower index of refraction as compared to a similar monomer hiving a relatively smaller alkyl group.

The alkylene group can generally be any divalent organic hydrocarbon group. The alkylene group can be straight or branched, and preferred alkylene groups can contain from 1 to 12 carbon atoms, more preferably from 2 to 6 carbons. The size and chemical structure of the alkylene group can affect the physical properties of the monomer and a polymer prepared therefrom, including the refractive index and viscosity of the monomer and the refractive index and Tg of a polymer prepared from the monomer. A relatively larger alkylene group can result in a monomer or polymer having a relatively lower index of refraction as compared to an otherwise similar monomer having a relatively smaller alkylene group. Relatively larger or more branched alkylene groups can provide a monomer which when polymerized has a relatively lower Tg compared to a polymer prepared from otherwise similar monomers having relatively smaller or less branched alkylene groups.

Bromine substitution can affect the index of refraction of the monomer. It is generally understood that bromine increases the index of refraction of the monomer. Bromine can be substituted on the aromatic portion of the monomer in any available amount or position, and will preferably be present in an amount to provide a monomer having a relatively high index of refraction, preferably at least 1.50. This can be accomplished, for example, by having at least two bromines directly attached to the aromatic ring.

Often, the position of the bromine can be a function of the materials and process used to prepare the brominated monomer (e.g., as described *infra).* Also, the position of an alkyl group on the aromatic ring can affect at least in part the position of bromines attached directly to the aromatic ring. If the alkyl group is at the 2 position (ortho-), bromines are preferably at the 4 and 6 positions.

The (alkyl, bromo)phenoxy alkyl (meth)acrylate monomers have the structure of formula (1) 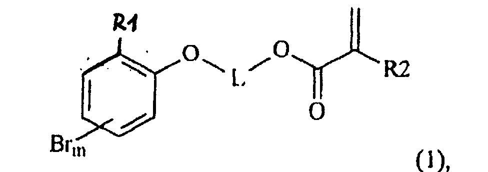 wherein:
R2 can be hydrogen (-H) or methyl (-CH3);
m is from 1 to 4, and is preferably 2;
L can be a straight chain or branched alkylcne group, preferably containing from 1 to 12 carbon atoms, more preferably from 2 to 6 carbon atoms; and
R1 can be a straight or branched alkyl having at least 2 carbon atoms, preferably having at least 3 and up to 12 carbon atoms.

The monomer preferably exhibits desired properties of index of refraction, melting point, and viscosity. The monomer preferably exhibits an index of refraction of at least 1.50. The melting point of the monomer can be below 60°C, preferably below 35°C or 30°C, and most preferably the monomer exists as a liquid at or near normal room temperature. The monomer can have a room temperature viscosity that allows the monomer or a polymerizable composition thereof to be processed, e.g., pumped, circulated, extruded, coated, formed, cured, or otherwise handled, at or near room temperature. Although viscosities outside of the following ranges can be useful, preferred viscosities of the monomer can be in the range from about 20 to 5000 mPa·s (centipoise (cps)), more preferably from 50 to 1000 mPa·s (cps), as measured at 23°C. Also, preferred monomers can be polymerized or copolymerized to provide polymeric materials having relatively low Tg, e.g., below 50°C. Particularly preferred monomers have both a relatively high index of refraction (e.g., greater than 1.50), and can produce a polymer having a relatively low Tg (e.g., below 50°C).

Useful monomers of the invention include monomers wherein R1 is located ortho to the phenoxy oxygen, as illustrated by formula 1: 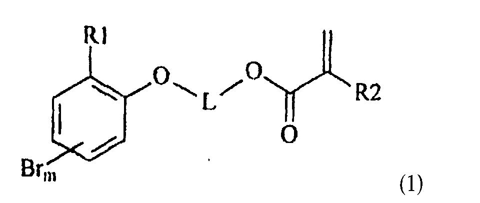 In formula (1), R2, m, L, and R 1 are as defined *supra*. In a particularly preferred embodiment, bromine atoms are located at the 4 and 6 positions on the phenoxy ring, ortho and para to the phenoxy oxygen atom, as illustrated by formula (2):

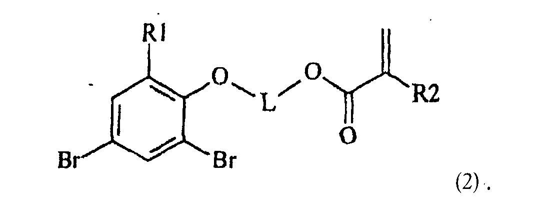

Particularly preferred monomers of formula (2) include 4,6-dibromo-2-alkyl phenoxy alkylene (meth)acrylates wherein the R1 alkyl has from 3 to 4 carbons, including monomers of the types shown in formulas (3) and (4), wherein R2 and L, are as defined above : 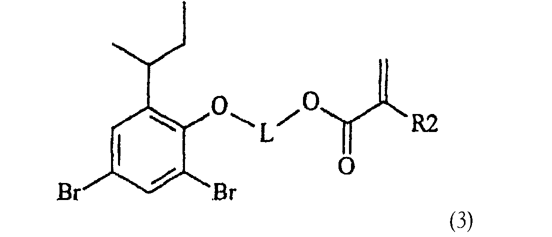 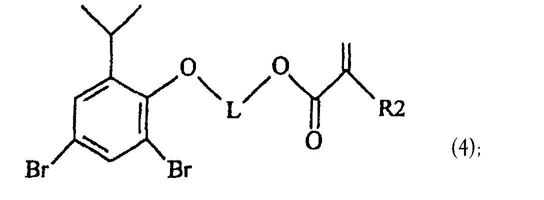 With R2 as hydrogen and 1. as ethylene these become 2-(4,6-dibromo-2-sec-butyl phenoxy) ethyl acrylate: 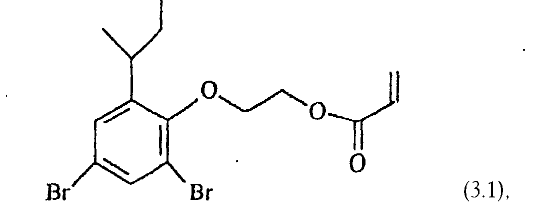 and 2-(4,6-dibromo-2-isopropyl phenoxy)ethyl acrylate: 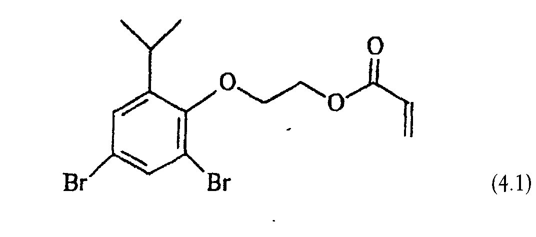 With R2 as hydrogen and L as hexylene these become 6-(4,6-dibromo-2-sec-butyl Phenoxy) hexyl acrylate: 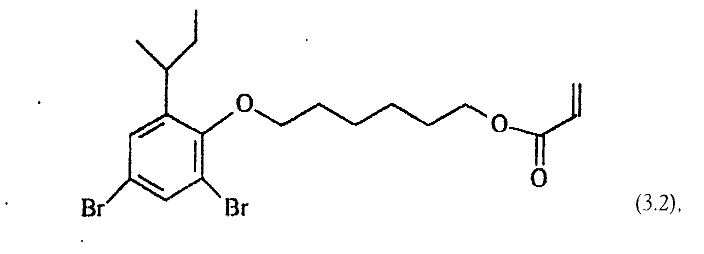 and 6-(4,6-dibromo-2-isopropyl phenoxy) hexyl acrylate:

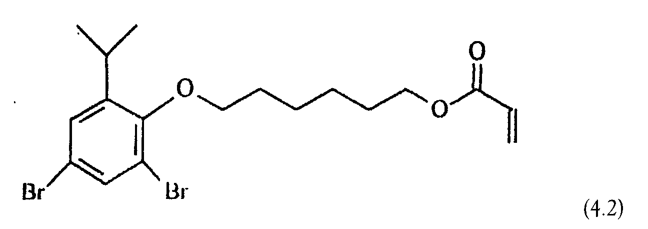

(Alkyl,bromo)phenoxy alkyl (meth)acrylate monomers of the invention can be prepared by methods generally useful in preparing substituted (e.g., brominated) phenoxy compounds and (meth)ncrylate monomers. Such methods are well known in the organic chemistry art.

As an example of one method of preparing the monomers of the invention, the following steps can be used. First, an alkyl-substituted phenol (alkylphenol) can be brominated to produce a brominated alkylphenol, as desired to prepare the desired brominated monomer. 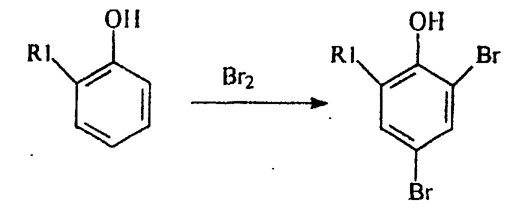 Alkylphenols are commercially available from Schenectady International Inc., Chemical Division, Schenectady, NY. Alkylphenols can be brominated by methods that are known in the organic chemistry art, and as described, for example, in the Kirk-Othmer Encyclopedia of Chemical Technology, Volume 4, 543 (4^{th} ed. 1992).

The brominated alkylphenol can be alkylated by known methods to produce an (alkyl,bromo)phenoxy alkanol compound. 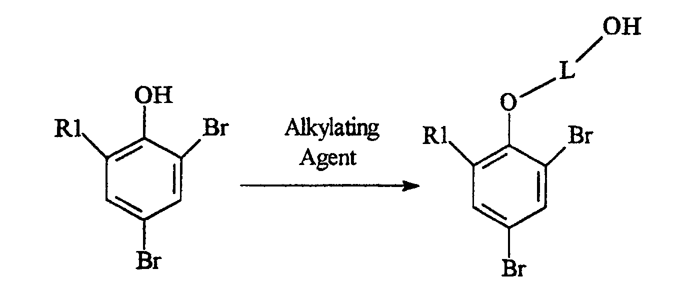 Alkylation methods are known in the chemical art, and are generally accomplished by introducing an alkylating agent, for example any one of an alkylene carbonate (e.g., ethylene carbonate), a chloroalkanol (e.g., chloroethanol) or an alkylene oxide (e.g., ethylene oxide) to the brominated alkylphenol under proper conditions to allow the alkylating agent to react with the phenol alcohol and cause alkylation. See, e.g., United States Patent No. 2,448,767; and Kirk-Othmer, Encyclopedia of Chemical Technology, Vol. 6, 146 (4^{th} ed. 1992).

Also using methods known in the organic chemistry art, the resulting (alkyl,bromo)phenoxy alkanol compound can be esterified to give a brominated (meth)acrylate monomer: 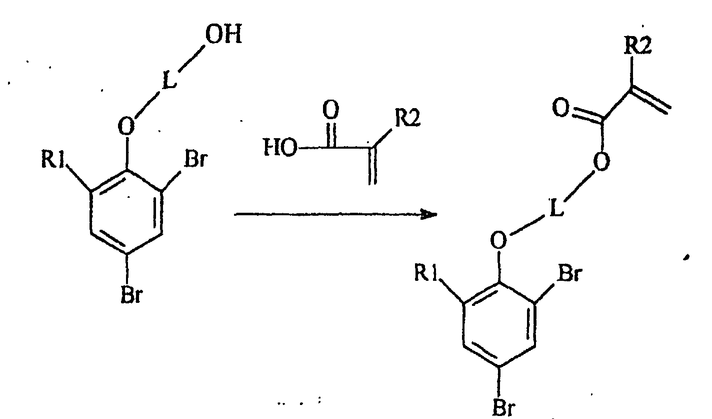 Esterification reactions are well known in the chemical art, and are described, for example, in the Kirk-Othmer Encyclopedia of Chemical Technology, vol. 1, 291 (4^{th} ed. 1992).

A preferred step in the preparation of the brominated monomer can be a purification step. Purification can be accomplished by any method known in the organic chemistry art, including methods of chromatography and distillation. For some brominated monomers, for example those that might suffer from thermal breakdown at elevated temperatures, it can be preferred to purify the monomers using ultra-high vacuum continuous distillation methods. These processes can be accomplished at pressures in the range from 0.1 to 133 Pa (1 to 1000 µm (micron) mercury (Hg)), and temperatures in the range from 100 to 200°C.

The brominated monomer of the invention, alone or in combination with other materials such as other unsaturated polymerizable comonomers, can be included in a polymerizable composition that can be polymerized or co-polymerized to produce useful polymers or copolymers. As used within the present description the term "polymerizable" refers to chemical compounds such as monomers and oligomers, etc., and chemical compositions, capable of polymerizing or copolymerizing (e.g., via unsaturated moieties) to produce a higher molecular weight material such as an oligomer, polymer, prepolymer, or polymeric material. The terms "polymer" and "polymeric material" arc used interchangeably to refer to materials prepared from the reaction of one or more polymerizable materials, e.g., one or more polymerizable monomer, oligomer, polymer, or prepolymer, etc. to produce a dimer, trimer, oligomer, copolymer, homopolymers, etc.

Useful comonomers to be reacted with acrylic monomers such as the brominated monomer described herein are known in the organic chemistry art, and can include any of a number of known and useful polymerizable moieties, e.g., vinyl, (meth)acrylate, N-vinyl, acrylic acid, methacrylic acid, allyl, acrylamide, acrylonitrile, etc. The comonomer can be mono- or multifunctional with respect to the unsaturated moiety, and where multifunctional, the unsaturated moieties need not be of identical chemistry.

Specific types of comonomer useful in the polymerizable composition can include the class of (meth)acrylate-functional comonomers such as butyl (meth)acrylate, as well as vinyl comonomers such as methyl styrene. The particular comonomers included in any given polymerizable composition, their molecular weight or weights, and the included amounts of each, can be chosen according to various factors such as the desired nature and properties of the polymerizable composition and the desired properties of the polymer or polymeric material to be prepared therefrom (e.g., index of refraction, glass transition temperature, melting point, viscosity, etc., of the polymerizable composition or polymeric material).

The polymerizable composition can also contain other ingredients that, as will be appreciated by those skilled in the art of polymeric materials, can be useful in such a polymerizable composition. For example, the polymerizable composition might contain a crosslinking agent, one or more surfactants, pigments, fillers, polymerization inhibitors, or other ingredients that can be useful within a polymerizable composition or an optical product. Such ingredients can be included in the composition in amounts known to be effective for their respective purposes.

A crosslinking agent can be useful to increase the glass transition temperature of the polymer resulting from crosslinking the polymerizable composition. Glass transition temperature of a composition can be measured by methods known in the art, such as Differential Scanning Calorimetry (DSC), modulated DSC (MDSC), or Dynamic Mechanical Analysis (DMA).

Polymeric beads, inorganic fillers, and/or pigments can be added to the polymerizable composition in order to improve processing, to impart slip and scratch resistance to the polymerized material, or to affect optical properties of the polymerized material. Examples of useful polymeric beads include those made of polystyrene, polyacrylatea, copolymers of styrene and acrylates, polyethylene, polypropylene, polytetrafluoroethylene, or combinations thereof Examples of inorganic fillers and pigments include solid or hollow glass beads, silica, zirconia, aluminum trihydroxide, and titanium dioxide.

The polymerizable composition can preferably have a room temperature viscosity that allows the polymerizable composition to be processed, e.g., pumped, circulated, extruded, coated, formed, cured, or otherwise handled, at or near room temperature. Although viscosities outside of the following ranges can also be useful, preferred viscosities of the polymerizable composition can be in the range from 20 to 5000 mPa·s (centipoise (cps)), more preferably in the range from 50 to 1000 mPa·s (cps), as measured at 23°C.

Polymerization of the composition can be accomplished by known and usual means, such as heating in the presence of a free-radical initiator, irradiation with electromagnetic radiation such as ultraviolet or visible light in the presence of suitable photoinitiators, and by electron beam. For reasons of convenience and production speed, a preferred method of polymerization might be by irradiation with ultraviolet or visible light in the presence of photoinitiator.

Polymeric materials (i.e., homopolymers or copolymers) prepared from the brominated monomer can exhibit a relatively low Tg, e.g., below 50°C.

the embodiment of the present invention are summerized in the following items:
1. An (Alkyl,bromo)phenoxy alkyl (meth)acrylate monomer comprising a (meth)acrylate, a phenoxy ring comprising alkyl and bromine substitution, and a divalent alkylene connecting the aliphatic ester (meth)acrylatc and the phenoxy ring according to Claim 1.
2. Monomer of item 1 having an index of refraction of at least 1.50.
3. Monomer of item 1 having a melting point below 60°C.
4. Monomer of item 1 which exists as a liquid at 25°C.
5. Monomer of item 1 having a viscosity in the range from 50 to 2000 mPa·s (centipoise) measured at 23°C.
6. Monomer of item 1 having an index of refraction of at least 1.50 and a viscosity in the range from 50 to 2000 mPa·s (centipoise) measured at 23°C.
7. Monomer of item 1 wherein the alkylene has from 1 to 12 carbons.
8. Monomer of item 1 wherein the alkyl group has from 3 to 12 carbons.
9. Monomer of item 1 wherein the alkyl group has 3 or 4 carbons.
10. Monomer of item 1 wherein the alkyl group is a branched alkyl having 3 or 4 carbons.
11. Monomer of item 1 chosen from 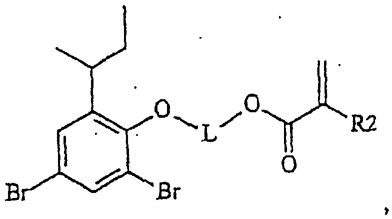 and 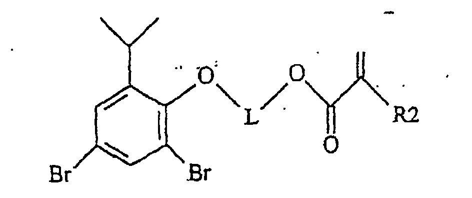 wherein L contains from 1 to 12 carbons.
12. Monomer of item 11 wherein L contains from 2 to 6 carbons.
13. Monomer of item 1 chosen from 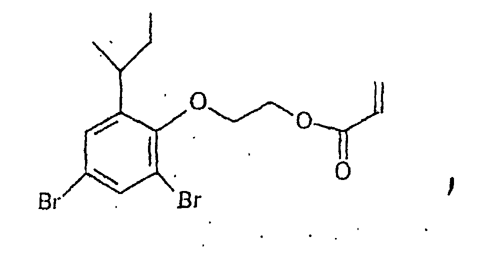 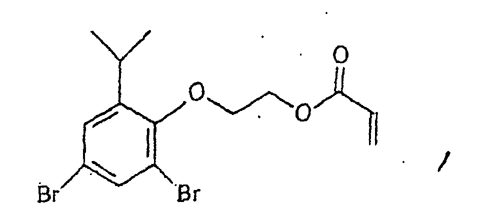 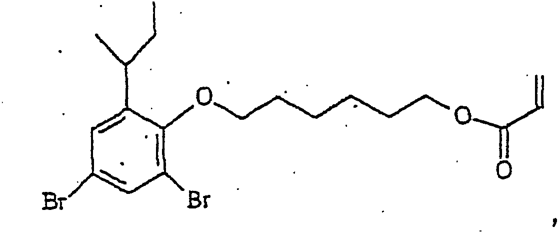 and 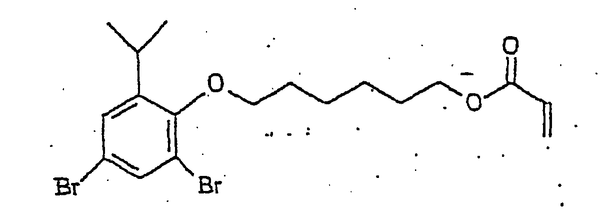
14. Monomer of item 1 chosen from
   2-(4,6-dibromo-2-sec-butyl phenoxy) ethyl acrylatc;
   6-(4,6-dibromo-2-isopropyl phenoxy) hexyl acrylate;
   2-(4,6-dibromo-2-sec-butyl phenoxy)-2-methyl ethyl acrylate;
   2-(4,6-dJbromo-2-sec-butyl phenoxy)-1-methyl ethyl acrylate;
   6-(4,6-dibromo-2-sec-butyl phenoxy) hexyl acrylatc;
   2-(4,6-dibromo-2-isopropyl phenoxy) ethyl acrylate ;
   6-(4,6-dibromo-2-dodecyl phenoxy) hexyl acrylate;
   2-(4,6-dibromo-2-dodecyl phenoxy) ethyl acrylate;
   6-(4,6-dibromo-2-sec-butyl phenoxy) hexyl methacrylate;
   6-(4,6-dibromo-2-isopropyl phenoxy) hexyl methacrylate;
   2-(4,6-dibromo-2-sec-butyl phenoxy) ethyl methacrylatc;
   2-(4,6-dibromo-2-isopropyl phenoxy) ethyl methacrylate;
   2-(4,6-dibromo-2-dodecyl phenoxy) ethyl methacrylate;
   and mixtures thereof.
15. A polymerizable composition comprising monomer of item 1 to 14.
16. The polymerizable composition of item 15 wherein the composition is liquid at room temperature.
17. The polymerizable composition of item 15 wherein the composition has a room temperature viscosity that allows the composition to be processed at room temperature.
18. The polymerizable composition of item 15 having a viscosity in the range from about 20 to 5000 mPa·s (centipoise).
19. The polymerizable composition of item 15 having an index of refraction of at least 1.50.
20. Polymer comprising a segment of the formula: 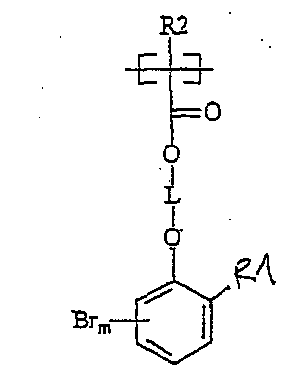 wherein m is from 1 to 4, R2 is -H or alkyl; R1 is a straight or branched alkyl, and L is a straight or branched alkylene.
21. Polymer of item 20 prepared from the monomer of item 1.
22. Polymer of item 20 wherein the polymer has a Tg below 50°C.
The invention will be more fully appreciated with reference to the following non-limiting examples in which the reoclion components are given as grams used or as weight percents (wt %), based on the total weight of (he reaction mixtures which are nominally 100 weight %. Dimensions in English units are nominal and conversion to metric units is approximate.

### Example 1: Synthesis of 2-(4,6-dibromo-2-sec-butyl phenoxy) ethyl acrylate.

### A. Preparation of 4,6-dibromo-2-sec-butyl phenol (DBsBP) (bromination)

In a 12 liter round bottom flask equipped with a mechanical stirrer, condenser, nitrogen cap, addition funnel and temperature probe, 1500 g (grams) of 2-sec-butylphenol was mixed with 4500 g of deionized water. The mixture was stirred with a mechanical mixer and purged with nitrogen for about 10 minutes. 3319 g bromine was added to the mixture drop-wise through the addition funnel. The temperature was maintained at 30°C or less using an ice bath. Following addition of the bromine, the reaction mixture was stirred for one hour at room temperature. Reaction completion was determined by gas chromatography, by monitoring the disappearance of the starting material, 2-sec-butylphenol, and of monobrominated species.

Upon completion of the reaction, 3960 g of ethyl acetate was added. The mixture wits stirred for 15 minutes and then allowed to phase split. The bottom (aqueous) layer was removed and 2686 g of a 13 wt% aqueous sodium hydrosulfite solution was added. The mixture was stirred well and then allowed to phase split. The bottom (aqueous) layer was removed and 2760 g of a 15 wt% aqueous sodium carbonate solution was added. The mixture was stirred well and then allowed to phase split. The bottom (aqueous) layer was removed and solvent was stripped from the top-layer using a rotary evaporator. This procedure provided approximately 2647 g of DBsBP.

### B. Preparation of 2- (4,6-dibromo-2-sec-butyl phenoxy) ethanol (alkylation)

A 500 ml round bottom flask was equipped with a magnetic stirrer, condenser and temperature probe. 40 g of the 4, 6-dibromo-2-sec-butylphenol, 12.5 g ethylene carbonate and 13.1 g triethylamine were added to the flask. The mixture was heated to reflux (∼120°C) and held at that temperature for about 24 hours. At this point, gas chromatograph analysis showed only 0.9% residual starting material, so the reaction was cooled to room temperature, 170 g t-butyl methyl ether was added, then 20.1 g of 37% HCI in 150 g of DI water was added. The mixture was shaken well and allowed to phase split and the lower aqueous phase removed. The mixture was then washed with a solution of 150 g water and 15 g of sodium carbonate and the lower aqueous phase was removed. The solvent was remove using a rotary evaporator to yield 40 grams of dark intermediate product. This product batch distilled using a 163°C pot, 115°C overhead condenser and 26.7 Pa (0.2mm Hg) vacuum to yield the yellow desired product, 2-(4,6-dibromo-2-sec-butyl phenoxy) ethanol.

### C. Preparation of 2-(4,6-dibromo-2-sec-butyl phenoxy) ethyl acrylate (esterificafion)

A 500 ml round bottom flask was equipped with a mechanical stirrer, Dean-Stark trap, condenser, and temperature probe. 25 g of 2-(4,6-dibromo-2-sec-butylphenoxy)ethanol, 125 g of toluene, 0.58 g of p-toluene sulfonic acid, 5.5 g of acrylic acid and -200 ppm each of methyl hydroquinone and hydroquinone were mixed together in the flask. The mixture was heated to reflux to azeotrope out the water generated during esterification. After 5 hours, gas chromatography analysis showed the reaction to be substantially complete (>98%), The reaction mixture was cooled washed three times: first with a solution of HCl in water, then with a solution of Na₂CO₃ in water and finally wills a solution of NaCI in water and the toluene was then stripped in vacuo. The product was purified using continuous distillation on a rolled Film evaporator (available from UIC Inc. of Joliet, 1L) at the following conditions: 0.1 Pa (1 micron Hg) vacuum and 130°C to obtain the product with >98% purity by NMR.

### Example 2: Preparation of 6-(4, 6-dihromo-2-isopropyl phenoxy) hexyl acrylate

### A. Preparation of 4,6-dibromo-2-isopropyl phenol (DBiPP) (bromination)

The procedure describing the preparation of DBsBP was followed using 1400 g of.2-isopropylphenol instead of the 2-sec-butylphenol, 4630 g of water, 3417 g of bromine, 4075 g of ethyl acetate, 2765 g of 13% (w/w) aqueous sodium hydrosulfite and 2842 g of 15% (w/w) aqueous sodium carbonate to produce 2556 g of DBiPP.

### B. Preparation of 6-(4,6-dibromo-2-isopropyl phenoxy) hexanol (alkylation)

A 12 liter, four neck, round bottom flask was set up with a mechanioal stirrer, condenser, temperature probe and addition funnel in a cooling bath. 800 grams of4,6-dibromo-2-isopropyl phenol (DBiPP) was added to the flask along with 4902 grams of deionized water and 408 grams of sodium iodide. Using the addition funnel, 435 grams of a 50% sodium hydroxide solution was added while maintaining the temperature below 25°C. The cooling bath was then removed and the reaction mixture was heated to reflux (100°C). Using a clean addition funnel, . 744 grams of 6-chlorohexanol was added over 1 hour and 30 minutes. The reaction was mixed two more hours at which point gas chromatography (GC) analysis indicated 0.3 % of the starting DBiPP remained unreacted. The solution was cooled and led at room temperature (22-25°C) overnight.

4196 grams of ethyl acetate was added to the reaction flask and mixed for 10 minutes (t-butyl methyl ether or other suitable organic solvent may be used). The mixture was allowed to phase split. The bottom aqueous layer was removed by vacuum and the pH was recorded at 11. The washing step was repeated a second time using a solution of 27 grams of 37% HCl in 980 grains of deionized water. The aqueous phase that was removed had a pH of 1. The washing step was repeated a third time using 980 grams of a 3% (w/w) aqueous sodium carbonate solution. Again the aqueous phase was removed and the pH was recorded at 11. The final washing was done with a 4.7%.(w/w) aqueous solution of sodium chloride (982 grains). The aqueous phase was again removed by vacuum. The organic phase filtered and concentrated on a rotary evaporator using a water aspirator. Residual solvent was removed using a vacuum pump while stirring the concentrate with a magnetic stirrer. 1250 grains of a yellow liquid was obtained. The yellow liquid was purified by continuous distillation using a rolled film evaporator. First 6-chlorohexanol and 6-iodohexanol were removed at the following conditions: 130°C oil bath and 0.6-2.7 Pa (5-20 microns Hg) vacuum. The residue was then continuously distilled on the rolled film evaporator using the following conditions: 130C oil bath and 0.1 Pa (1 micron Hg) vacuum. 832 grams of the water white alkylated product (6-(4,6-dibromo-2-isopropyl phenoxy) hexanol) was recovered. It can be noted here that optionally, a wiped film evaporator can be used in place of the rolled film evaporator.

### C. Preparation of 6-(4,6-dibromo-2-isopropyl phenoxy) hexyl acrylate (esterification)

A five liter, four neck round bottom flask was equipped with a mechanical stirrer, Dean Stark trap, condenser and temperature probe. The flask was charged with 600 grams of 6-(4,6-dibromo-2-isopropyl phenoxy) hexanol; 2805 grams of toluene; ~200 ppm each of methyl hydroquinone and hydroquinone; 15.2 grams p-toluene sulfonic acid and 13 I grams acrylic acid. This mixture was heated to reflux with stirring to azeotrope the water. After six hours of refluxing, 30 ml of water had been removed and 9y.2% of the 6-(4,6-dibromo-2-isopropyl phenoxy) hexanol had been converted to the 6-(4,6-dibromo-2-isopropyl phenoxy) hexyl acrylate based on gas chromatography (GC) analysis. The solution was then cooled and " allowed to mix overnight.

828 grams of a 0.27% HCI.solut.!on was added to the reaction flask and mixed for five minutes. The mixture was allowed to phase split and the aqueous bottom phase (pH=1) was removed by vacuum. The washing was repeated by adding 903 grams of an 8.9% (w/w) aqueous solution of sodium carbonate. The aqueous phase was removed after phase separation. A third wash was done using 867 grams of a 5.1% (w/w) aqueous sodium chloride solution. The aqueous phase. was again removed by vacuum. The organic phase was filtered and concentrated on a rotary evaporator using a waler aspirator. Residual solvent was removed using a vacuum pump while stirring the concentrate with a magnetic stirrer. 650 grams of a hazy, light yellow liquid was obtained. The yellow liquid was then purified by continuous distillation in a rolled film evaporator using the following conditions: 175°C oil bath and 0.1 Pa (1 micron Hg) vacuum to give the water white product. NMR analysis indicated a 98.8% purity prior to distillation and a purity of>99% in the distilled product,. 6-(4,6-dibromo-2-iso-propyl phenoxy) hexyl acrylate.

### Example 3:Preparation of a mixture of 2-(4, 6-dibromo-2-sec-butyl phenoxy) 2-methyl ethyl acrylate and 2-(4,6-dibromo-2-sec-butyl phenoxy) 1-methyl ethyl acrylale.

### A. Preparation of 4,G-dibromo-2-sec-butyl phenol (DBsBP) (bromination)

4,6-dibromo-2-sec-butyl phenol (DBsBP) was prepared according to the procedure described in Example 1,

### B. Preparation of a mixture of 2-(4,6-dibromo-2-sec-butyl phenoxy) 2-methyl ethanol and 2-(4,6-dibromo-2-sec-butyl phenoxy) 1-methyl ethanol (alkylation)

A 500 ml round bottom llask was equipped willi a magnetic stirrer, condenser and a temperature probe. The flask was charged with 60 grams of 4,6-dibromo-2-sec-butyl phenol (DBsBP), 21.9 grams of propylene carbonate, and 19.7 grams of triethylamine. The mixture was heated to reflux (120°C) and held at that temperature for about 24 hours The mixture was cooled to room temperature and the flask was charged with 170 grams of t-butyl methyl ether. 170 grams of a 4.3% aqueous solution of HCl was added to the reaction flask and mixed. The mixture was allowed phase separate and the aqueous phase was removed. 165 grams of a 9.1% aqueous solution of sodium carbonate was then added to the flask, mixed and allowed to phase separate. The aqueous phase was again removed. The ether solvent was then removed using a rotary evaporator. 68 grams of dark alkylated product was recovered. ¹³C NMR analysis indicated the recovered product to be predominately a mixture of 2-(4, 6-dibromo-2-sec-butyl phenoxy) 2-methyl ethanol and 2-(4, 6-dibromo-2-sec-butyl phenoxy) 1-methyl ethanol.

### C. Preparation of a mixture of 2-(4,6-dibromo-2-sec-butyl phenoxy)-2-methyl ethyl acrylate and 2-(4,6-dibromo-2-sec-butyl phenoxy)-1-methyl ethyl acrylate (esterification)

A five liter, four neck round bottom flask was equipped with a mechanical stirrer, Dean Stark trap, condenser and temperature, probe. The flask was charged with 25 grams of the intermediate alkylated product from example 3B, 125 grams of toluene, 5.9 grams of acrylic acid, 0.58 grams p-toluene sulfonic acid, and about 200 ppm each of methyl hydroquinone and hydroquinone. The mixture was heated to reflux to azeotrope out the water generated during esterification. Gas. chromatography analysis after three hours of reflux showed only slight conversion. The toluene was then stripped using a rotary evaporator and an equal amount of xylenes were added back to the flask. This mixture was then heated to reflux (∼140°C) to azeotrope out water. After one hour, gas chromatography showed 33%.conversion of the alcohol. Acrylic acid (2 grams) was added to the flask and reflux was continued an additional two hours. At this point, the conversion was >90%. The reaction mixture was then cooled and washed as described in Section 1C above, and the xylenes were stripped using a rotary evaporator. The crude product remaining in the evaporator flask was passed through a flash chromatography column using methylene chloride to isolate the desired product. The solvent was again removed using a rotary evaporator. Residual solvent was removed using a vacuum pump while stirring the concentrate with a magnetic stirrer. ¹³C NMR analysis of the product showed a mixture of 2-(4,6-dibromo-2-sec-butyl phenoxy)-2-methyl ethyl acrylate (∼77%) and 2-(4,6-dibromo-2-sec-butyl phenoxy)-1-methyl ethyl acrylate(∼11%).

### Examples 4-12

Using bromination, alkylation and esterification steps similar to those described in Examples I to 3, a variety of(alkyl,bromo)phenoxy alkyl (meth)acrylate monomers were prepared. By using appropriate starting materials, stoichiometric quantities, and the methods described in Examples 1-3, one skilled in the art of organic chemistry could prepare the materials of Examples 4-12 outlined in Table 1.

**Table 1**

| Synthesized Monomers | |
|---|---|
| **Example** | **Name** |
| 1 | 2-(4,6-dibromo-2-sec-butyl phenoxy) ethyl acrylate |
| 2 | 6-(4,6-dibromo-2-isopropyl phenoxy) hexyl acrylate |
| 3 | 2-(4,6-dibromo-2-sec-butyl phenoxy)-2-methyl ethyl acrylate and 2-(4,6-dibromo-2-sec-butyl phenoxy)-1-methyl ethyl acrylate ate |
| 4 | 6-(4,6-dibromo-2-sec-butyl phenoxy) hexyl acrylate |
| 5 | 2-(4,6-dibromo-2-isopropyl phenoxy) clhyl acrylale |
| 6 | 6-4,6-dibromo-2-dodecyl phenoxy) hexyl acrylate |
| 7 | 2-(4,6-dibromo-2-dodccyl phcnoxy) ethyl acrylate |
| 8 | 6-(4,6-dibromo-2-sec-butyl phenoxy) hexyl methacrylate |
| 9 | 6-(4,6-dibromo-2-isopropyl phenoxy) hexyl methacrylate |
| 10 | 2-(4,6-dibromo-2-sec-butyl phenoxy) ethyl methacrylate |
| 11 | 2-(4,6-dibromo-2-isopropyl phenoxy) ethyl methacrylate |
| 12 | 2-(4,6-dibromo-2-clodecyl phenoxy) ethyl methacrylate |

### Experimental Methods

Homopolymerization - Homopolymers of several or the above noted monomers were prepared by combining 2 grams of monomer with 10 grams of ethyl acetate solvent and 0.006 grams of Vazo 64 or Vazo 88 initiatior (available from Dupont) in a 4 ounce (118.3 ml) glass bottle. The bottle was purged for one minute with nitrogen at a flow rate or one liter per minute. The bottle was sealed and then placed in a rotating water bath at 55°C (65°C if Vazo 88 initiator was used) for 24 hours to effect essentially complete polymerization. The homopolymer was recovered for Tg analysis by evaporating the solvent at 105°C.

Glass Transition (Tg) - Glass transition temperatures (Tg) were determined using a differential scanning calorimeter (DSC-7) manufactured by Perkin Elmer, Norwalk, X CT. A 10 mg polymer sample was heated at a rate of 20°C/minute, cooled at a rate of 40°C/minute and then reheated at 20°C/minute. The Tg was calculated on the second.heating cycle.

Viscosity - Steady shear viscosity measurements were-made at 25°C using a 40 mm parallel plate fixture on Rheometrics Stress Rheometer (DSR) which is a commercially available instrument sold by Rheometrics, Scientific, One Possumtown Rd., Piscataway, NJ 08854. The sample of monomer was sandwiched between the two plates and squeezed out till a gap setting of 0.45 to 0.5 mm was achieved. The excess material was.cleaned out and the sample was then subjected to a set of predetermined shear rates. At each shear rate, the viscosity measurement was made when the torque was at steady state. Analysis indicated the viscosity to be independent of the shear rate. The viscosity is reported in Table 2 in mPa.s (centipoise (cps))

Refractive Index - The refractive index of resin compositions and cured films were measured using an Abbe Refractometer, made by Erma Inc. of Tokyo Japan and distributed by Fisher Scientific.

**Table 2**

| Properties-of Monomers and Homopolymers | | | |
|---|---|---|---|
| **Example** | **Monomer Refractive Index** | **Monomer Viscosity, m Pa·s (cps)** | **Tg of Homonolymer (°C)** |
| 1 | 1.5455 | 120 | 33 |
| 2 | 1.5340 | 90 | -10 |
| 3 | 1.5432 | | |
| 4 | 1.5265 | 90 | -20 |
| 5 | 1.5490 | 120 | |
| 6 | 1.5135 | BC) | |
| 7 | 1.5210 | 80 | |
| 8 | 1.5300 | ∼100 | |
| 9 | 1.5335 | ∼100 | 7 |
| 10 | 1.5460 | 120 | 39 |
| 11 | 1.5470 | 120 | |
| 12 | 1.5120 | Solid | |

## Claims

1. A monomer of the formula: 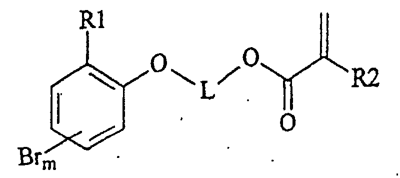 wherein m is from 1 to 4, R2 is -H or methyl, R1 is a straight or branched alkyl having at least 2 carbons, and L is a straight chain or branched alkylene.

2. The monomer of claim 1 selected from the group consisting of 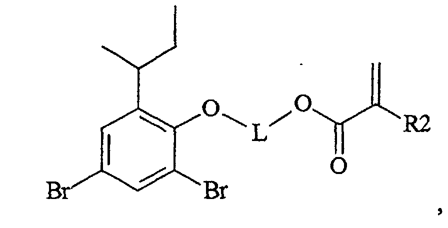 and 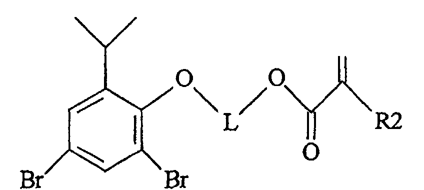 wherein L contains from 1 to 12 carbons, and R2 is hydrogen or a methyl group.

3. The monomer of claim 1 selected from the group consisting of 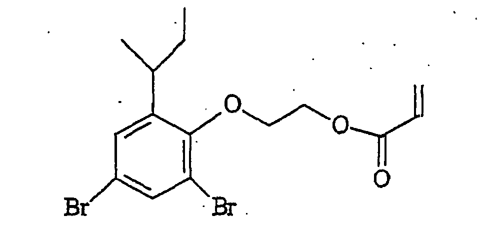 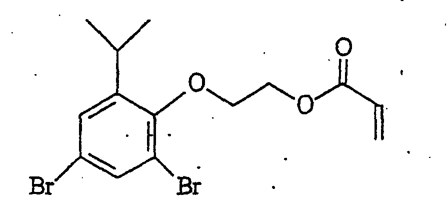 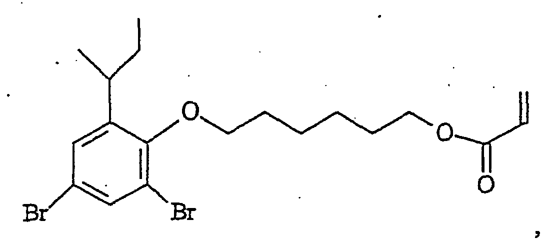 and

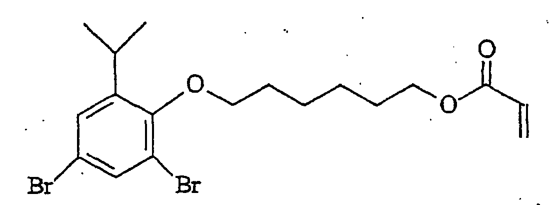

4. A monomer selected from the group consisting of:
2-(4,6-dibromo-2-sec-butyl phenoxy) ethyl acrylate;
6-(4,6-dibromo-2-isopropyl phenoxy) hexyl acrylate;
2-(4,6-dibromo-2-sec-butyl phenoxy)-2-methyl ethyl acrylate;
2-(4,6-dibromo-2-sec-butyl phenoxy)-1-methyl ethyl acrylate;
6-(4,6-dibromo-2-sec-butyl phenoxy) hexyl acrylate;
2-(4,6-dibromo-2-isopropyl phenoxy) ethyl acrylate;
6-(4,6-dibromo-2-dodecyl phenoxy) hexyl acrylate,
2-(4,6-dibromo-2-dodecyl phenoxy) ethyl acrylate;
6-(4,6-dibromo-2-sec-butyl phenoxy) hexyl methacrylate;
6-(4,6-dibromo-2-isopropyl phenoxy) hexyl methacrylate;
2-(4,6-dibromo-2-sec-butyl phenoxy) ethyl methacrylate;
2-(4,6-dibromo-2-isopropyl phenoxy) ethyl methacrylate;
2-(4,6-dibromo-2-dodecyl phenoxy) ethyl methacrylate;
and mixtures thereof.

5. A polymerizable composition comprising a monomer according to any of claims 1 to 4.

6. A polymer comprising a segment of the formula: 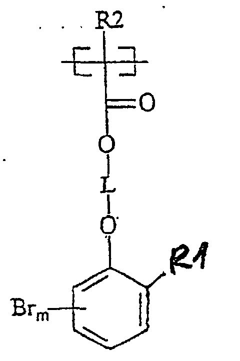 wherein m is from 1 to 4, R2 is -H or alkyl, R1 is a straight or branched alkyl, and L is a straight or branched alkylene.

7. The polymer of claim 6 obtainable from the monomer of any of claims 1 to 4.

## Patentansprüche

1. Monomer der Formel 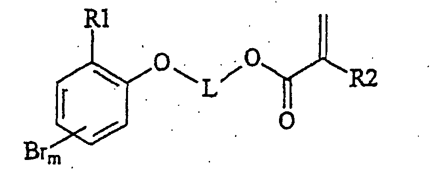 wobei m 1 bis 4 ist, R2 ein Wasserstoffatom oder eine Methylgruppe bedeutet, R1 einen linearen oder verzweigten Alkylrest mit mindestens 2 Kohlenstoffatomen bedeutet, und L einen geradkettigen oder verzweigten Alkylenrest bedeutet.

2. Monomer nach Anspruch 1, ausgewählt aus 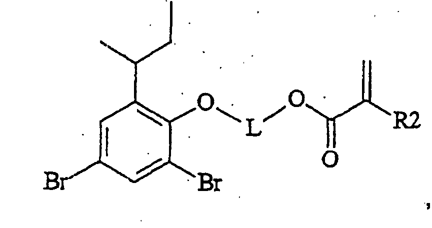 und 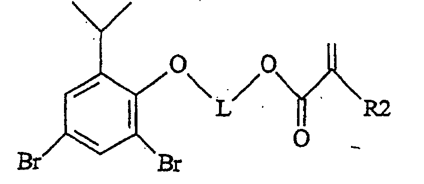 wobei L 1 bis 12 Kohlenstoffatome enthält und R2 ein Wasserstoffatom oder eine Methylgruppe bedeutet.

3. Monomer nach Anspruch 1, ausgewählt aus 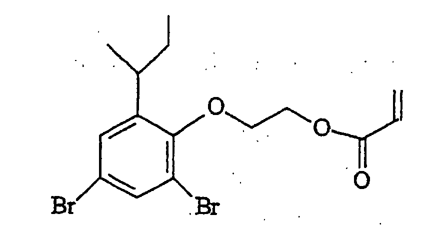 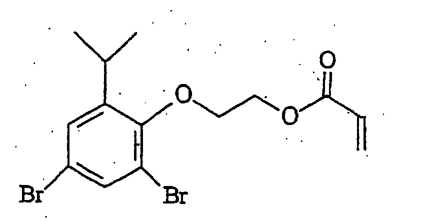 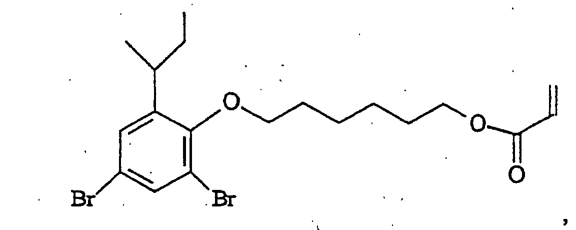 und

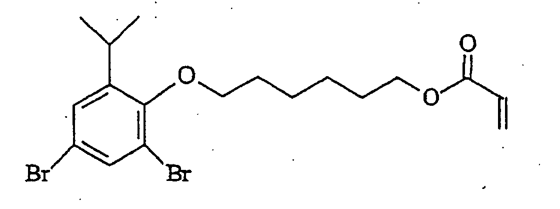

4. Monomer, ausgewählt aus:
2-(4,6-Dibrom-2-sek-butylphenoxy)ethylacrylat;
6-(4,6-Dibrom-2-isopropylphenoxy)hexylacrylat;
2-(4,6-Dibrom-2-sek-butylphenoxy)-2-methylethylacrylat;
2-(4,6-Dibrom-2-sek-butylphenoxy)- 1 -methylethylacrylat;
6-(4,6-Dibrom-2-sek-butylphenoxy)hexylacrylat;
2-(4,6-Dibrom-2-isopropylphenoxy)ethylacrylat;
6-(4,6-Dibrom-2-dodecylphenoxy)hexylacrylat;
2-(4,6-Dibrom-2-dodecylphenoxy)ethylacrylat;
6-(4,6-Dibrom-2-sek-butylphenoxy)hexylmethacrylat;
6-(4,6-Dibrom-2-isopropylphenoxy)hexylmethacrylat;
2-(4,6-Dibrom-2-sek-butylphenoxy)ethylmethacrylat;
2-(4,6-Dibrom-2-isopropylphenoxy)ethylmethacrylat;
2-(4,6-Dibrom-2-dodecylphenoxy)ethylmethacrylat
und Gemischen davon.

5. Polymerisierbare Zusammensetzung, umfassend ein Monomer nach einem der Ansprüche 1 bis 4.

6. Polymer, umfassend ein Segment der Formel: 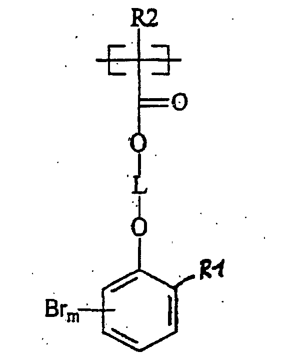 wobei m 1 bis 4 ist, R2 ein Wasserstoffatom oder einen Alkylrest bedeutet, R1 einen linearen oder verzweigten Alkylrest bedeutet und L einen linearen oder verzweigten Alkylenrest bedeutet.

7. Polymer nach Anspruch 6, erhältlich aus dem Monomer eines der Ansprüche 1 bis 4.

## Revendications

1. Monomère de formule : 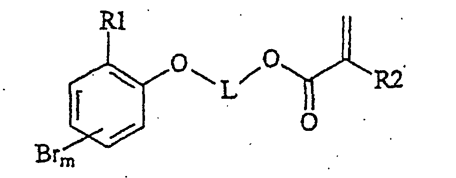 dans laquelle m est entre 1 et 4, R2 est -H ou méthyle, R1 est un groupe alkyle à chaîne linéaire ou ramifiée ayant au moins 2 carbones, et L est un groupe alkylène à chaîne linéaire ou ramifiée.

2. Monomère selon la revendication 1 1 choisi dans le groupe constitué de 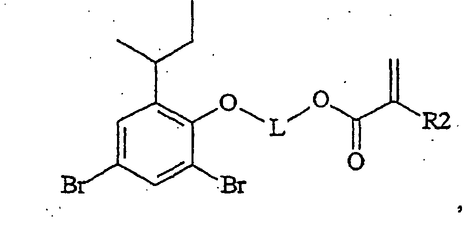 et 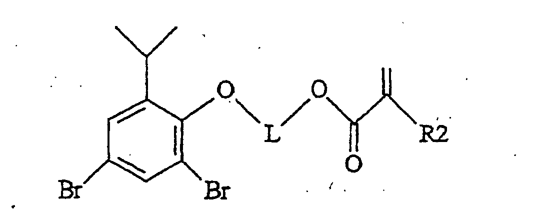 dans lesquelles L contient de 1 à 12 atomes de carbone, et R2 est un hydrogène ou un groupe méthyle.

3. Monomère selon la revendication 1 choisi dans le groupe constitué de 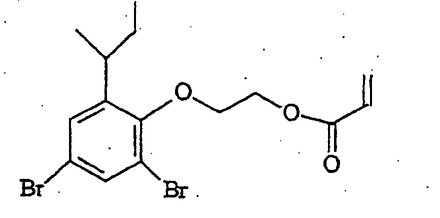 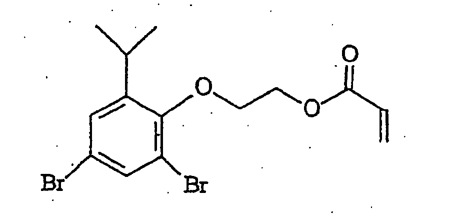 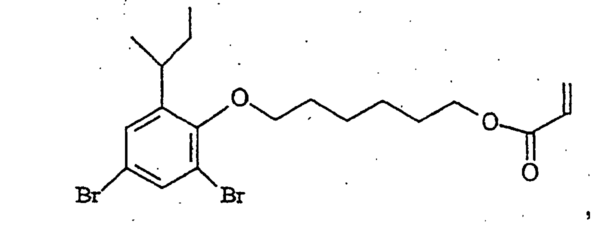 et

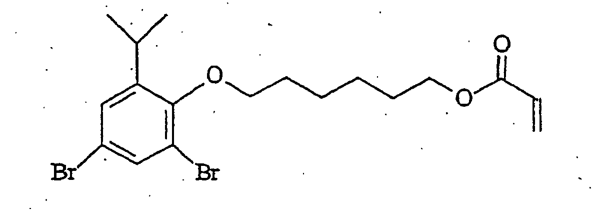

4. Monomère choisi dans le groupe constitué de :
acrylate de 2- (4,6-dibromo-2-sec.-butylphénoxy)éthyle ;
acrylate de 6.-(4,6-dibromo-2-isopropylphénoxy)hexyle ;
acrylate de 2-(4,6-dibromo-2-sec.-butylphénoxy)-2-méthyl-éthyle ;
acrylate de 2-(4,6-dibromo-2-sec.-butylphénoxy)-1-méthyl-éthyle ;
acrylate de 6-(4,6-dibromo-2-sec.-butylphénoxy)hexyle ;
acrylate de 2-(4,6-dibromo-2-isopropylphénoxy)éthyle ;
acrylate de 6-(4,6-dibromo-2-dodécylphénoxy)hexyle ;
acrylate de 2-(4,6-dibromo-2-dodécylphénoxy)éthyle ;
méthacrylate de 6-(4,6-dibromo-2-sec.-butylphénoxy)-hexyle ;
méthacrylate de 6-(4,6-dibromo-2-isopropylphénoxy)-hexyle ;
méthacrylate de 2-(4,6-dibromo-2-sec.-butylphénoxy)-éthyle ;
méthacrylate de 2-(4,6-dibromo-2-isopropylphénoxy)-éthyle ;
méthacrylate de 2- (4, 6-dibromo-2-dodécylphénoxy)-éthyle ; et leurs mélanges.

5. Composition polymérisable comprenant un monomère selon l'une quelconque des revendications 1 à 4.

6. Polymère comprenant un segment de formule : 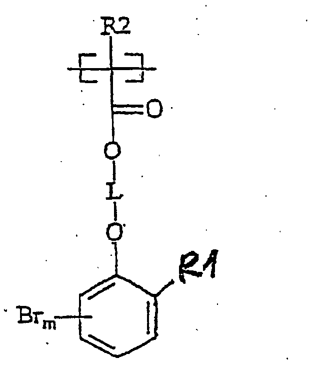 dans laquelle m est entre 1 et 4, R2 est -H ou un groupe alkyle, R1 est un groupe alkyle à chaîne linéaire ou ramifiée, et L est un groupe alkylène à chaîne linéaire ou ramifiée.

7. Polymère selon la revendication 6 susceptible d'être obtenu à partir du monomère de l'une quelconque des revendications 1 à 4.
